# EUROPEAN PATENT SPECIFICATION

(11) **EP 2 635 249 B1**
(45) Date of publication and mention of the grant of the patent: **18.01.2017**
(21) Application number: 11776172.6
(22) Date of filing: 28.10.2011
(51) Int. Cl.: A61K 8/02, A61K 8/04, A61K 8/19, A61K 8/22, A61K 8/41, A61Q 5/10, A61K 8/20

(54) **COLOURING MOUSSE COMPOSITION BASED ON AN ALKANOLAMINE AND AN AMMONIUM SALT**
SCHAUMFÖRMIGE FÄRBEZUSAMMENSETZUNG ENTHALTEND EINE ALKANOLAMINE UND EIN AMMONIUMSALZ
COMPOSITION DE COLORATION MOUSSE A BASE D'ALKANOLAMINE ET DE SEL D'AMMONIUM

(30) Priority: 12.11.2010 US 412969 P; 02.11.2010 FR 1059015
(43) Date of publication of application: 11.09.2013
(73) Proprietor: L'Oréal, 75008 Paris (FR)
(72) Inventor: GOGET, Caroline, F-75018 Paris (FR); SABOURIN, Richard, F-75005 Paris (FR); ALLARD, Delphine, F-75009 Paris (FR); ASCIONE, Jean-Marc, F-75003 Paris (FR)
(74) Representative: Fevrier, Murielle Françoise E.
(86) International application number: PCT/EP2011/068931
(87) International publication number: WO 2012/059411

(56) References cited:
- EP-A1- 2 204 160
- GB-A- 1 271 331
- DATABASE GNPD [Online] MINTEL; October 2010 (2010-10), "Awa Color Color Selection Hair Colourant", XP002643788, Database accession no. 1419847
- DATABASE GNPD [Online] MINTEL; July 2010 (2010-07), "Men's Soft Colouring Foam", XP002643787, Database accession no. 1379126
- DATABASE GNPD [Online] MINTEL; October 2010 (2010-10), "Bubble Hair Color", XP002644315, Database accession no. 1408790
- DATABASE GNPD [Online] MINTEL; June 2010 (2010-06), "Soft Colouring Foam Dark Tone", XP002644316, Database accession no. 1360388
- DATABASE GNPD [Online] MINTEL; October 2010 (2010-10), "Mousse Hair Colourant", XP002644135, Database accession no. 1460247

## Description

The present invention pertains to a method for colouring hair, in mousse form, employing a composition comprising oxidation dye precursors, and also to a composition in mousse from.

The methods of colouring human keratinic fibres, such as the hair, include permanent or oxidation dyeing. More particularly, this dyeing method uses one or more oxidation dyes, usually one or more oxidation bases optionally combined with one or more couplers.

In general, oxidation bases are chosen from *ortho-* or *para*-phenylenediamines, *ortho-* or *para*-aminophenols and heterocyclic compounds. These oxidation bases are colourless or weakly coloured compounds, which, when combined with oxidizing products, can give access to coloured species.

The shades obtained with these oxidation bases are often varied by combining them with one or more couplers, these couplers being chosen especially from aromatic *meta*-diamines, *meta*-aminophenols, *meta*-diphenols and certain heterocyclic compounds, such as indole compounds.

The variety of molecules used as oxidation bases and couplers allows a wide range of colours to be obtained.

Permanent dyeing processes therefore involve employing, together with the dyeing composition, an aqueous composition comprising at least one oxidizing agent such as hydrogen peroxide, under alkaline pH conditions in the great majority of cases. The alkaline agent conventionally used is aqueous ammonia, or other alkaline agents, such as alkanolamines.

EP 2 204 160 A1 discloses a two-part dye comprising Lauramidopropyl betaine, ethanolamine and ammonium hydrogen-carbonate, at least one oxidizing agent and at least one oxidation dye precursor.

The colouring compositions may take various forms, such as lotions, gels, emulsions, creams or mousses. Colouring mousses are pleasant to use, but are often subject to poor persistence. It is possible, for example, to observe rapid disappearance of the mousse following application, or inconsistent application along the fibres.

There is a genuine need to develop oxidation dyeing compositions in mousse form which are easy to prepare and to apply and which remain sufficiently stable over time while retaining effective dyeing properties.

This aim and others are achieved by the present invention, which accordingly provides a composition for colouring human keratinic fibres such as the hair, in mousse form, comprising:
(a) at least one amphoteric surfactant;
(b) at least one alkanolamine;
(c) ammonium chloride;
(d) at least one oxidizing agent,
(e) at least one oxidation dye precursor.

The invention also relates to a method for colouring human keratinic fibres, employing this composition.

The invention likewise provides a multi-compartment device comprising in one of the compartments a composition containing one or more amphoteric surfactants, one or more oxidation dye precursors, one or more alkanolamines, and ammonium chloride, and, in a second compartment, a composition containing one or more oxidizing agents and a distributor of mousse based on the composition of the invention obtained by mixing the two preceding compositions.

The invention further provides a device for colouring keratinic fibres, capable of forming a mousse as described above, comprising the composition of the invention in liquid form and a mousse distributor for delivering the composition in the form of a mousse.

The composition of the invention takes the form of a mousse which is particularly pleasant to apply. It has a light and airy texture, so making such mousses particularly pleasant to use. The qualities of the mousse are sufficiently persistent to allow the colouring product to be applied uniformly and without running. The composition of the invention makes it possible to conserve dyeing properties such as the strength of the colour, the resistance to external agents (shampoos, perspiration, light) and selectivity that are particularly effective.

Other features and advantages of the invention will emerge more clearly from a reading of the description and examples which follow.

In the text hereinbelow, unless otherwise indicated, the limits of a range of values are included in that range. The term "at least one" in combination with an ingredient of the composition signifies "one or more".

The human keratinic fibres treated by the method according to the invention are preferably the hair.

The composition comprises one or more alkanolamines. The term "alkanolamine" means an organic amine comprising a primary, secondary or tertiary amine function, and one or more linear or branched C₁-C₈ alkyl groups bearing one or more hydroxyl radicals.

Alkanolamines such as mono-, di- or tri- alkanolamines comprising from one to three identical or different C₁-C₄ hydroxyalkyl radicals are in particular suitable for performing the invention.

Compounds of this type include monoethanolamine, diethanolamine, triethanolamine, monoisopropanolamine, diisopropanolamine, N-dimethylaminoethanolamine, 2-amino-2-methyl-1-propanol, triisopropanolamine, 2-amino-2-methyl-1,3-propanediol, 3-amino-1,2-propanediol, 3-dimethylamino-1,2-propanediol and trishydroxymethylaminomethane. The alkanolamine is preferably monoethanolamine.

The composition according to the invention generally comprises an amount of alkanolamine varying from 0.01% to 30% by weight, preferably from 0.1 % to 20% by weight relative to the weight of the said composition.

The composition of the invention advantageously has an amount of ammonium chloride ranging from 0.01% to 30% by weight, preferably from 0.5% to 10% by weight relative to the weight of the said composition.

According to one particular embodiment, the composition of the invention may comprise organic alkaline agents other than alkanolamines and ammonium salts, such as aqueous ammonia, sodium hydroxide, potassium hydroxide, ethoxylated and/or propoxylated ethylenediamines, amino acids and the compounds of formula (I) below: 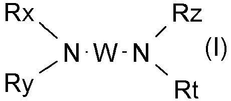 in which W is a C₁-C₆ alkylene residue optionally substituted with a hydroxyl group or a C₁-C₆ alkyl radical; Rx, Ry, Rz and Rt, which may be identical or different, represent a hydrogen atom or a C₁-C₆ alkyl, C₁-C₆ hydroxyalkyl or C₁-C₆ aminoalkyl radical.

Examples of such amines that may be mentioned include 1,3-diaminopropane, 1,3-diamino-2-propanol, spermine and spermidine.

As amino acids that may be used in the present invention, mention may be made especially of aspartic acid, glutamic acid, alanine, arginine, ornithine, citrulline, asparagine, carnitine, cysteine, glutamine, glycine, histidine, lysine, isoleucine, leucine, methionine, N-phenylalanine, proline, serine, taurine, threonine, tryptophan, tyrosine and valine.

According to one preferred embodiment, the composition of the invention contains a small amount of aqueous ammonia; preferably the composition of the invention does not contain aqueous ammonia.

The composition according to the invention further comprises one or more oxidizing agents.

The oxidizing agents are selected for example from hydrogen peroxide, urea peroxide, alkali metal bromates or ferricyanides, peroxygenated salts such as, for example, persulphates, perborates, peracids and precursors thereof, and the percarbonates of alkali metals or alkaline earth metals. Advantageously, the oxidizing agent is hydrogen peroxide.

The amount of oxidizing agent(s) represents more particularly from 0.1% to 20% by weight, preferably from 0.5% to 10% by weight, relative to the weight of the composition.

As indicated earlier on, the composition according to the invention comprises one or more oxidation dye precursors.

Oxidation dye precursors which can be used include oxidation bases and couplers.

As an example, the oxidation bases are selected from para-phenylenediamines, bisphenylalkylenediamines, para-aminophenols, ortho-aminophenols, heterocyclic bases and their addition salts.

The para-phenylenediamines include, for example, para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylene-diamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-β-hydroxyethyl-para-phenylenediamine, N-(β,γ-di-hydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine, N-(β-methoxyethyl)-para-phenylenediamine, 4-aminophenylpyrrolidine, 2-thienyl-para-phenylenediamine, 2-β-hydroxy-ethylamino-5-aminotoluene, 3-hydroxy-1-(4'-aminophenyl)pyrrolidine and their addition salts with an acid.

Among the para-phenylenediamines mentioned above, para-phenylenediamine, para-tolylenediamine, 2-isopropyl-para-phenylenediamine, 2-β-hydroxyethyl-para-phenylenediamine, 2-β-hydroxyethyloxy-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine and 2-β-acetylaminoethyloxy-para-phenylenediamine, and the addition salts thereof with an acid, are particularly preferred.

Among the bis(phenyl)alkylenediamines that may be mentioned, for example, are N,N'-bis(β-hydroxyethyl)-N,N'-bis(4'-aminophenyl)-1,3-diaminopropanol, N,N'-bis-(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine, 1,8-bis(2,5-diaminophenoxy)-3,6-dioxaoctane, and the addition salts thereof.

Among the para-aminophenols that may be mentioned, for example, are para-aminophenol, 4-amino-3-methylphenol, 4-amino-3-fluorophenol, 4-amino-3-chlorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

Among the ortho-aminophenols that may be mentioned, for example, are 2-aminophenol, 2-amino-5-methylphenol, 2-amino-6-methylphenol and 5-acetamido-2-aminophenol, and the addition salts thereof.

Among the heterocyclic bases that may be mentioned, for example, are pyridine derivatives, pyrimidine derivatives and pyrazole derivatives.

The pyridine derivatives include the compounds described, for example, in patents GB 1 026 978 and GB 1 153 196, such as 2,5-diaminopyridine, 2-(4-methoxy-phenyl)amino-3-aminopyridine, 3,4-diaminopyridine and their addition salts.

Other pyridine oxidation bases that are useful in the present invention are the 3-aminopyrazolo[1,5-a]pyridine oxidation bases or addition salts thereof described, for example, in patent application FR 2 801 308. Examples include pyrazolo[1,5-a]pyridin-3-ylamine; 2-acetylaminopyrazolo-[1,5-a]pyridin-3-ylamine; 2-morpholin-4-ylpyrazolo[1,5-a]pyridin-3-ylamine; 3-aminopyrazolo[1,5-a]pyridine-2-carboxylic acid; 2-methoxypyrazolo[1,5-a]pyridin-3-ylamine; (3-amino-pyrazolo[1,5-a]pyridin-7-yl)methanol; 2-(3-aminopyrazolo[1,5-a]pyridin-5-yl)ethanol; 2-(3-aminopyrazolo[1,5-a]pyridin-7-yl)ethanol; (3-aminopyrazolo[1,5-a]pyridin-2-yl)-methanol; 3,6-diaminopyrazolo[1,5-a]pyridine; 3,4-diaminopyrazolo[1,5-a]pyridine; pyrazolo[1,5-a]pyridine-3,7-diamine; 7-morpholin-4-ylpyrazolo[1,5-a]pyridin-3-ylamine; pyrazolo[1,5-a]pyridine-3,5-diamine; 5-morpholin-4-ylpyrazolo[1,5-a]pyridin-3-ylamine; 2-[(3-aminopyrazolo[1,5-a]pyridin-5-yl)-(2-hydroxyethyl)amino]ethanol; 2-[(3-amino-pyrazolo[1,5-a]pyridin-7-yl)-(2-hydroxyethyl)amino]ethanol; 3-amino-pyrazolo[1,5-a]-pyridine-5-ol; 3-aminopyrazolo[1,5-a]pyridin-4-ol; 3-aminopyrazolo[1,5-a]pyridin-6-ol; 3-aminopyrazolo[1,5-a]pyridin-7-ol; and also their addition salts.

Among the pyrimidine derivatives that may be mentioned are the compounds described, for example, in the patents DE 2359399; JP 88-169571; JP 05-63124; EP 0770375 or patent application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine, 2,5,6-triaminopyrimidine and their addition salts and their tautomeric forms, when a tautomeric equilibrium exists.

Among the pyrazole derivatives that may be mentioned are the compounds described in the patents DE 3843892, DE 4133957 and patent applications WO 94/08969, WO 94/08970, FR-A-2 733 749 and DE 195 43 988, such as 4,5-diamino-1-methylpyrazole, 4,5-diamino-1-(β-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-*tert*-butyl-1-methylpyrazole, 4,5-diamino-1-*tert*-butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethyl-pyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-methylaminopyrazole, 3,5-diamino-4-(β-hydroxyethyl)amino-1-methylpyrazole, and their addition salts. 4,5-Diamino-1-(β-methoxyethyl)pyrazole may also be used.

A 4,5-diaminopyrazole will preferably be used, and even more preferentially 4,5-diamino-1-(β-hydroxyethyl)pyrazole and/or a salt thereof.

Pyrazole derivatives that may also be mentioned include diamino-N,N-dihydro-pyrazolopyrazolones and especially those described in patent application FR-A-2 886 136, such as the following compounds and the addition salts thereof: 2,3-diamino-6,7-dihydro-1H,5H-pyrazolo[1,2-a]pyrazol-1-one, 2-amino-3-ethylamino-6,7-dihydro-1H,5H-pyrazolo[1,2-a]pyrazol-1-one, 2-amino-3-isopropylamino-6,7-dihydro-1H,5H-pyrazolo[1,2-a]pyra-zol-1-one, 2-amino-3-(pyrrolidin-1-yl)-6,7-dihydro-1H,5H-pyrazolo[1,2-a]pyrazol-1-one, 4,5-diamino-1,2-dimethyl-1,2-dihydropyrazol-3-one, 4,5-diamino-1,2-diethyl-1,2-dihydropyrazol-3-one, 4,5-diamino-1,2-di-(2-hydroxyethyl)-1,2-dihydropyrazol-3-one, 2-amino-3-(2-hydroxyethyl)amino-6,7-dihydro-1 H,5H-pyrazolo[1,2-a]pyrazol-1-one, 2-amino-3-dimethylamino-6,7-dihydro-1H,5H-pyrazolo[1,2-a]pyrazol-1-one, 2,3-diamino-5,6,7,8-tetrahydro-1H,6H-pyridazino[1,2-a]-pyrazol-1-one, 4-amino-1,2-diethyl-5-(pyrrolidin-1-yl)-1,2-dihydropyrazol-3-one, 4-amino-5-(3-dimethylaminopyrrolidin-1-yl)-1,2-diethyl-1,2-dihydropyrazol-3-one, 2,3-diamino-6-hydroxy-6,7-dihydro-1H,5H-pyrazolo[1,2-a]pyrazol-1-one. Preference is given to using 2,3-diamino-6,7-dihydro-1H,5H-pyrazolo[1,2-a]pyrazol-1-one and/or a salt thereof.

4,5-Diamino-1-(β-hydroxyethyl)pyrazole and/or 2,3-diamino-6,7-dihydro-1H,5H-pyrazolo[1,2-a]pyrazol-1-one and/or a salt thereof will preferentially be used as heterocyclic bases.

The couplers which can be used in the composition of the invention include in particular meta-phenylenediamines, meta-aminophenols, meta-diphenols, naphthalenic couplers, heterocyclic couplers and their addition salts.

Examples include 1,3-dihydroxybenzene, 1,3-dihydroxy-2-methylbenzene, 4-chloro-1,3-dihydroxybenzene, 2,4-diamino-1-(ß-hydroxyethyloxy)benzene, 2-amino-4-(ß-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene, 2,6-bis(β-hydroxyethyl-amino)toluene, 6-hydroxyindoline, 2,6-dihydroxy-4-methylpyridine, 1-H-3-methyl-pyrazol-5-one, 1-phenyl-3-methylpyrazol-5-one, 2,6-dimethylpyrazolo[1,5-b]-1,2,4-triazole, 2,6-dimethylpyrazolo[3,2-c]-1,2,4-triazole, 6-methylpyrazolo[1,5-a]-benzimidazole, their addition salts with an acid, and mixtures thereof.

The addition salts of the oxidation bases and couplers are especially chosen from the addition salts with an acid such as the hydrochlorides, hydrobromides, sulfates, citrates, succinates, tartrates, lactates, tosylates, benzenesulfonates, phosphates and acetates.

The oxidation base or bases are generally present each in an amount of between 0.0001% to 10% by weight relative to the total weight of the composition, and preferably from 0.005% to 5% by weight relative to the total weight of the composition.

The coupler or couplers each represent in general from 0.0001% to 10% by weight relative to the total weight of the emulsion, and preferably from 0.005% to 5% by weight relative to the total weight of the composition.

The composition according to the invention may contain cationic or nonionic, natural or synthetic direct dyes.

Examples of particularly suitable direct dyes include the nitro dyes of the benzene series; direct azo dyes; azomethine dyes; methine dyes; azocarbocyanines such as tetraazacarbocyanines (tetraazapentamethines); direct quinone dyes and more particularly anthraquinone, naphthoquinone or benzoquinone dyes; direct azine dyes; xanthene dyes; triarylmethane dyes; indoamine dyes; indigoid dyes; phthalocyanines; porphyrins and natural direct dyes, alone or in mixtures. In particular, mention may be made of direct dyes from among: azo; methine; carbonyl; azine; nitro (hetero)aryl; tri(hetero)arylmethane; porphyrin; phthalocyanine and natural direct dyes, alone or as mixtures.

When present, the direct dye or dyes represent more particularly from 0.0001% to 10% by weight of the total weight of the composition, and preferably from 0.005% to 5% by weight.

The composition according to the invention comprises one or more amphoteric surfactants.

The amphoteric or zwitterionic surfactant or surfactants which can be used in the present invention may be, in particular, optionally quaternized derivatives of secondary or tertiary aliphatic amines in which the aliphatic group is a linear or branched chain containing from 8 to 22 carbon atoms, the said amine derivatives containing at least one anionic group such as, for example, a carboxylate, sulphonate, sulphate, phosphate or phosphonate group. Particular mention may be made of alkyl (C₈-C₂₀) betaines, sulphobetaines, alkyl (C₈-C₂₀) amidoalkyl (C₁-C₆) betaines or alkyl (C₈-C₂₀) amidoalkyl (C₁-C₆) sulphobetaines, preferably alkyl (C₈-C₂₀) amidoalkyl (C₁-C₆) betaines. Among the optionally quaternized derivatives of secondary or tertiary aliphatic amines that can be used, as defined above, mention may also be made of the compounds of respective structures (II) and (III) below:

Ra-CONHCH₂CH₂-N+(Rb)(Rc)(CH₂COO-) (II)

in which:
Ra represents a C₁₀-C₃₀ alkyl or alkenyl group derived from an acid
Ra-COOH, preferably present in hydrolysed coconut oil, a heptyl, nonyl or undecyl group,
Rb represents a beta-hydroxyethyl group, and
Rc represents a carboxymethyl group;
and

Ra'-CONHCH₂CH₂-N(B)(B') (III)

in which :
B represents -CH₂CH₂OX',
B' represents -(CH₂)z-Y', with z = 1 or 2,
X' represents the group -CH₂-COOH, CH₂-COOZ', -CH₂CH₂-COOH, -CH₂CH₂-COOZ', or a hydrogen atom,
Y' represents -COOH, -COOZ', or the group -CH₂-CHOH-SO₃H or -CH₂-CHOH-SO₃Z',
Z' represents an ion derived from an alkali metal or alkaline-earth metal, such as sodium, an ammonium ion or an ion derived from an organic amine,
Ra' represents a C₁₀-C₃₀ alkyl or alkenyl group of an acid Ra'-COOH which is preferably present in coconut oil or in hydrolysed linseed oil, an alkyl group, more particularly a C₁₇ alkyl group in its iso form, or an unsaturated C₁₇ group.

These compounds are classed in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, cocoamphodipropionic acid.

By way of example, mention may be made of the cocoamphodiacetate sold by the company Rhodia under the trade name Miranol^{®} C2M Concentrate.

Among the abovementioned amphoteric or zwitterionic surfactants it is preferred to use (C8-20 alkyl)betaines such an cocoylbetaine, (C8-20 alkyl)amido(C2-8 alkyl)betaines such as cocoylamidopropylbetaine and mixtures thereof. More preferably, the amphoteric or zwitterionic surfactants are selected from cocoylmidopropylbetaine and cocoylbetaine.

According to one embodiment, the composition comprises at least one amphoteric surfactant, at least one anionic surfactant and at least one nonionic surfactant.

An "anionic surfactant" is a surfactant containing only anionic groups as ionic or ionizable groups. These anionic groups are selected preferably from the groups CO₂H, CO₂⁻, SO₃H, SO₃⁻, OSO₃H, OSO₃⁻, H₂PO₃ ,HPO₃⁻, PO₃²⁻, H₂PO₂, HPO₂, HPO₂⁻, PO₂⁻, POH and PO⁻.

As examples of anionic surfactants that may be used in the composition according to the invention, mention may be made of alkyl sulphates, alkyl ether sulphates, alkylamido ether sulphates, alkylaryl polyether sulphates, monoglyceride sulphates, alkyl sulphonates, alkylamide sulphonates, alkylaryl sulphonates, alphaolefin sulphonates, paraffin sulphonates, alkyl sulphosuccinates, alkyl ether sulphosuccinates, alkylamide sulphosuccinates, alkyl sulphoacetates, acyl sarcosinates, acyl glutamates, alkyl sulphosuccinamates, acyl isethionates and N-acyltaurates, salts of alkyl monoesters of polyglycoside-polycarboxylic acids, acyl lactylates, D-galactoside-uronic acid salts, alkyl ether carboxylic acid salts, alkylaryl ether carboxylic acid salts, alkylamido ether carboxylic acid salts; and the corresponding non-salified forms of all these compounds; the alkyl and acyl groups of all these compounds comprising from 6 to 24 carbon atoms and the aryl group denoting a phenyl group.

These compounds may be oxyethylenated and then preferably comprise from 1 to 50 ethylene oxide units.

The salts of C6-24 alkyl monoesters and polyglycoside-polycarboxylic acids may be selected from C6-24 alkyl polyglycoside-citrates, C6-24 alkyl polyglycoside-tartrates and C6-24 alkyl polyglycoside-sulphosuccinates.

When the anionic surfactant(s) (ii) are in salt form, they may be chosen from alkali metal salts such as the sodium or potassium salt and preferably the sodium salt, the ammonium salts, the amine salts and in particular amino alcohol salts or the alkaline-earth metal salts such as the magnesium salt.

Examples of salts of amino alcohols include in particular the salts of mono-, di- and triethanolamine, the salts of mono-, di- or triisopropanolamine, the salts of 2-amino-2-methyl-1-propanol, 2-amino-2-methyl-1,3-propanediol and tris(hydroxymethyl)aminomethane.

Alkali metal or alkaline-earth metal salts and in particular the sodium or magnesium salts are preferably used.

Among the anionic surfactants, it is preferred according to the invention to use the alkyl sulphate salts and alkyl ether sulphate salts and mixtures thereof.

The nonionic surfactants are more particularly selected from mono- or polyoxyalkylenated and mono- or polyglycerolated nonionic surfactants. The oxyalkylene units are more particularly oxyethylene or oxypropylene units or a combination thereof, preferably oxyethylene units.

Examples of oxyalkylenated nonionic surfactants that may be mentioned include:
- oxyalkylenated (C₈-C₂₄)alkylphenols,
- saturated or unsaturated, linear or branched, oxyalkylenated C₈-C₃₀ alcohols,
- saturated or unsaturated, linear or branched, oxyalkylenated C₈-C₃₀ amides,
- esters of saturated or unsaturated, linear or branched, C₈-C₃₀ acids and of polyethylene glycols,
- polyoxyethylenated esters of saturated or unsaturated, linear or branched, C₈-C₃₀ acids and of sorbitol,
- saturated or unsaturated, oxyethylenated plant oils,
- condensates of ethylene oxide and/or of propylene oxide, inter alia, alone or as mixtures;
the surfactants having between 1 and 100, preferably between 2 and 50 and more preferably between 2 and 30 mol ethylene oxide and/or propylene oxide.

In accordance with one preferred embodiment of the invention, the oxyalkylenated nonionic surfactants are selected from oxyethylenated C₈-C₃₀ alcohols containing from 1 to 100 mol of ethylene oxide; and polyoxyethylenated esters of linear or branched, saturated or unsaturated C₈-C₃₀ acids and sorbitol, containing from 1 to 100 mol ethylene oxide.

As examples of mono- or polyglycerolated nonionic surfactants it is preferred to use mono- or polyglycerolated C₈-C₄₀ alcohols.

In particular, the monoglycerolated or polyglycerolated C₈-C₄₀ alcohols correspond to the following formula:

RO-[CH₂-CH(CH₂OH)-O]ₘ-H

in which R represents a linear or branched C₈-C₄₀ and preferably C₈-C₃₀ alkyl or alkenyl radical, and m represents a number ranging from 1 to 30 and preferably from 1 to 10.

As examples of compounds that are suitable in the context of the invention, mention may be made of lauryl alcohol containing 4 mol of glycerol (INCI name: Polyglyceryl-4 Lauryl Ether), lauryl alcohol containing 1.5 mol of glycerol, oleyl alcohol containing 4 mol of glycerol (INCI name: Polyglyceryl-4 Oleyl Ether), oleyl alcohol containing 2 mol of glycerol (INCI name: Polyglyceryl-2 Oleyl Ether), cetearyl alcohol containing 2 mol of glycerol, cetearyl alcohol containing 6 mol of glycerol, oleocetyl alcohol containing 6 mol of glycerol, and octadecanol containing 6 mol of glycerol.

The alcohol may represent a mixture of alcohols in the same way that the value of m represents a statistical value, which means that, in a commercial product, several species of polyglycerolated fatty alcohols may coexist in the form of a mixture.

Among the monoglycerolated or polyglycerolated alcohols, it is more particularly preferred to use the C₈/C₁₀ alcohol containing 1 mol of glycerol, the C₁₀/C₁₂ alcohol containing 1 mol of glycerol and the C₁₂ alcohol containing 1.5 mol of glycerol.

Nonionic surfactants also include non-oxyethylenated esters of fatty acids and sorbitan, esters of fatty acids and sucrose, optionally oxyalkylenated alkylpolyglycosides, alkylglucoside esters, derivatives of N-alkylglucamine and of N-acylmethylglucamine, aldobionamides, and amine oxides.

According to one particular embodiment, the composition of the invention comprises one or more amphoteric surfactants, one or more nonionic surfactants and one or more anionic surfactants. According to this embodiment, the nonionic surfactant is preferably selected from saturated or unsaturated oxyethylenated fatty alcohols and saturated or unsaturated oxyethylenated plant oils. The anionic surfactant is selected from alkyl sulphates or alkyl ether sulphates.

The total amount of surfactants in the composition of the invention is generally between 0.1 % and 30% by weight, preferably from 1% to 20%, more preferably from 1% to 10%, by weight relative to the weight of the composition.

The composition may also comprise various adjuvants which are typically used in compositions for colouring or lightening hair, such as anionic, cationic or nonionic polymers or mixtures thereof; antioxidants; penetrants; sequestrants; fragrances; dispersants; film-formers; ceramides; preservatives; and opacifiers.

The above adjuvants are generally present in an amount, for each of them, of between 0.01 % and 20% by weight relative to the weight of the composition.

The composition according to the invention preferably comprises one or more cationic polymers.

The composition according to the invention may comprise water and/or one or more organic solvents.

Organic solvents include for example monoalcohols or diols, which are linear or branched, preferably saturated, and contain 2 to 10 carbon atoms, such as ethyl alcohol, isopropyl alcohol, hexylene glycol (2-methyl-2,4-pentanediol), neopentyl glycol and 3-methyl-1,5-pentanediol, butylene glycol, dipropylene glycol and propylene glycol; aromatic alcohols such as benzyl alcohol and phenylethyl alcohol; polyols having more than two hydroxyl functions, such as glycerol; polyol ethers such as, for example, the monomethyl, monoethyl and monobutyl ethers of ethylene glycol, propylene glycol or its ethers such as, for example, the monomethyl ether of propylene glycol; and also the alkyl ethers, especially C₁-C₄ alkyl ethers, of diethylene glycol, such as, for example, the monoethyl ether or monobutyl ether of diethylene glycol, alone or in a mixture.

The organic solvents, when they are present, generally represent between 1% and 40% by weight relative to the total weight of the dye composition, and preferably between 5% and 30% by weight relative to the total weight of the dye composition.

The composition is preferably aqueous. In this case it preferably comprises from 30% to 95% of water by weight, more preferably from 40% to 90% of water by weight, even more preferably from 50% to 85% of water by weight relative to the total weight of the composition.

The pH of the composition according to the invention, if it is aqueous, is generally between 3 and 12, preferably between 5 and 11, more preferably between 7 and 11, end points included.

The pH may be adjusted to the desired value by means of acidifying or alkalifying agents which are commonly used in dyeing keratinic fibres, and in particular the alkanolamines and the ammonium salts of the invention.

On application to the keratinic fibres, the composition according to the invention is in the form of a mousse.

The composition in mousse form according to the invention is formed from a mixture of air or an inert gas with the composition described above.

According to one particularly preferred embodiment, the composition according to the invention is in the form of a temporary mousse produced just before use.

According to this embodiment, the composition may be packaged in a mousse distributor. The compositions in question may be aerosol products, distributed from a pressurized container by means of a propellant gas, thus forming a mousse at the time of their distribution, or compositions which are distributed from a container by means of a mechanical pump connected to a distribution head, where the passage of the composition into the distribution head transforming it into a mousse no later than at the outlet orifice of such a head.

According to one first variant, the distributor may be an aerosol containing, in addition to the base composition, divided generally into two parts, one with the oxidizing agent or agents and the other with the dye precursor or precursors, a propellant gas. In a configuration of this kind, the two parts are generally stored each separately in a container under pressure. Accordingly, the propellant gases selected in each of the containers may be adapted to the component contained.

The propellant gas which can be used may be selected from carbon dioxide, nitrogen, nitrogen oxide, dimethyl ether, volatile hydrocarbons such as butane, isobutane, propane and pentane, and mixtures thereof.

In practice, for this variant, use will be made either of an aerosol pack with a single container containing two compartments internally, or a double aerosol, hence containing two containers. In both cases, the distribution head is such that the system sprayed in mousse form is the composition according to the invention, in other words the mixture of the composition with the oxidizing agent or agents and the composition with the oxidation dye precursor or precursors.

According to another embodiment, the composition may be located within a pump canister mousse distributor. These distributors comprise a distribution head for delivering the composition, a pump and a descender tube for transferring the composition from the container to the head for delivering the product. The mousse is formed by forcing the composition through a material comprising a porous substance, such as a sintered material, a metal or plastic filtering grid, or similar structures.

Distributors of these kinds are well known to the skilled person and are described in U.S. Pat No. 3,709,437 (Wright), U.S. Pat. No. 3,937,364 (Wright), U.S. Pat No. 4,022,351 (Wright), U.S. Pat No. 4,1147,306 (Bennett), U.S. Pat No. 4,184,615 (Wright), U.S. Pat No. 4,598,862 (Rice), U.S. Pat No. 4,615,467 (Grogan et al.), and U.S. Pat No. 5,364,031 (Tamiguchi et al.).

In practice for this variant, the oxidizing agent or agents are packaged in a first container equipped with a stopper, and the oxidation dye precursor or precursors are packaged in a second container, distinct from the first, and likewise closed by a closing member. The closing member may be a pump distribution mechanism. The composition according to the invention is then formed by mixing, before use, a composition with the oxidizing agent or agents and a composition with the oxidation dye precursor or precursors. For this purpose, in order to limit the number of containers provided, one of the first and second containers defines an internal volume sufficient to accommodate the whole of the two compositions. The mixture of the compositions can be homogenized by closing this container and shaking it. The container is advantageously closed directly with the distribution head. This distribution head comprises a mechanical pump held in a band intended to be mounted, by snap-fastening or screwing, onto the neck of the container holding the mixture. The pump comprises a pump body connected to a descender tube for allowing the whole of the mixture to be distributed. The pump also comprises a pushbutton for actuating the pump body such that, on each actuation, one dose of composition is drawn into the descender tube and ejected in the form of a mousse at the distribution orifice of the head.

In this example, the containers are preferably made from a thermoplastic material, and are obtained by extrusion blow moulding or injection blow moulding processes. In particular, the container intended for packaging the composition with the oxidation dye precursor or precursors is made from a material having a non-zero proportion of EVOH. The pump is, for example, the standard "F2 - L9" model sold by Rexam.

According to this preferred embodiment, the invention provides a non-aerosol device comprising the composition of the invention.

The colouring method according to the invention involves applying the composition according to the invention to wet or dry human keratinic fibres for a time sufficient for development of the desired coloration. According to the invention, the composition applied to the keratinic fibres is in mousse from. The colouring method is generally employed at ambient temperature (between 15 to 25°C) and at temperatures which may be up to 60°C to 80°C.

After a contact time of a minute to an hour, preferably 5 minutes to 30 minutes, the keratinic fibres are rinsed with water and optionally undergo washing with a shampoo, followed by rinsing with water.

The examples below serve to illustrate the invention, though without having any limitative nature.

### EXAMPLES

The compositions below are prepared (the amounts are expressed in g% of active substances)

| **Composition** | **% by weight** |
|---|---|
| Oxidation dye precursors (base(s) and coupler(s)) | qs shade |
| Coco betaine | 1 |
| Sodium laureth sulphate | 1 |
| Monoethanolamine | 5 |
| Ammonium chloride | 1 |
| Sorbitol | 4 |
| POLYQUATERNIUM-6 | 0.5 |
| POLYQUATERNIUM-39 | 0.05 |
| 40-Oxyethylenated hydrogenated castor oil | 0.4 |
| Hydrogen peroxide | 4.5 |
| Glycerol | 3 |
| Cetearyl alcohol | 0.2 |
| Erythorbic acid | 0.2 |
| Sodium metabisulphite | 0.2 |
| Sodium salicylate | 0.021 |
| Tetrasodium pyrophosphate | 0.024 |
| Tetrasodium etidronate | 0.036 |
| EDTA | 0.08 |
| Phosphoric acid | |
| Water | qs 100 |

The composition above is obtained by mixing weight for weight, before use, two compositions, A and B, as follows:

| **Composition A** | **% by weight** |
|---|---|
| Oxidation dye precursors (base(s) and coupler(s)) | qs shade |
| Coco betaine | 2 |
| Monoethanolamine | 10 |
| Ammonium chloride | 2 |
| Sorbitol | 8 |
| POLYQUATERNIUM-6 | 1 |
| 40-Oxyethylenated hydrogenated castor oil | 0.8 |
| Erythorbic acid | 4 |
| Sodium metabisulphite | 4 |
| EDTA | 16 |
| Water | qs 100 |

| **Composition B** | **% by weight** |
|---|---|
| Sodium laureth sulphate | 2 |
| POLYQUATERNIUM-39 | 1 |
| Hydrogen peroxide | 9 |
| Glycerol | 6 |
| Cetearyl alcohol | 0.4 |
| Sodium salicylate | 0.042 |
| Tetrasodium pyrophosphate | 0.048 |
| Tetrasodium etidronate | 0.072 |
| Phosphoric acid | qs pH 2.2 |
| Water | qs 100 |

The mixture is introduced in an amount of 65 g (32.5 g of composition A + 32.5 g of composition B) into a pump canister (Rexam L9) equipped with a descender tube. The device, by pumping, allows a mousse to be obtained which is sufficiently compact to be applied to natural or permanent-waved grey hair containing 90% white hairs, without undergoing instantaneous disintegration. The comfort on application is very good. After 30 minutes of contact, the locks of hair are rinsed, washed with a standard shampoo, rinsed again and then dried to give the desired coloration. This coloration is strong and of low selectivity.

## Claims

1. Composition for colouring human keratinic fibres in mousse form, comprising:
(a) at least one amphoteric surfactant;
(b) at least one alkanolamine;
(c) ammonium chloride;
(d) at least one oxidizing agent;
(e) at least one oxidation dye precursor.

2. Composition according to Claim 1, further comprising an anionic surfactant.

3. Composition according to Claim 1 or 2, wherein amphoteric surfactant is selected from alkyl (C₈-C₂₀) betaines, sulphobetaines, alkyl (C₈-C₂₀) amidoalkyl (C₁-C₆) betaines or alkyl (C₈-C₂₀) amidoalkyl (C₁-C₆) sulphobetaines, preferably alkyl (C₈-C₂₀) amidoalkyl (C₁-C₆) betaines.

4. Composition according to any of the preceding claims, comprising one or more amphoteric surfactants, one or more nonionic surfactants and one or more anionic surfactants.

5. Composition according to any of Claims 1 to 4, wherein the surfactant or surfactants are present in a total amount of from 0.1% to 30% by weight, preferably from 1% to 20%, more preferably from 1% to 10%, by weight relative to the weight of the composition.

6. Composition according to any of the preceding claims, wherein the alkanolamine is monoethanolamine.

7. Composition according to any of the preceding claims, wherein the amount of alkanolamine varies from 0.01% to 30% by weight, preferably from 0.1 % to 20% by weight relative to the weight of the said composition.

8. Composition according to any of the preceding claims, wherein the amount of ammonium chloride varies between 0.01% to 30% by weight, preferably from 0.5% to 10% by weight relative to the weight of the said composition.

9. Composition according to any of the preceding claims, wherein the oxidizing agent is hydrogen peroxide.

10. Composition according to any of the preceding claims, comprising one or more oxidation bases and one or more couplers.

11. Aerosol device comprising a means of generating in mousse form a composition as defined in any of Claims 1 to 10.

12. Aerosol device according to Claim 11, comprising one container with 2 compartments, or comprising two containers.

13. Non-aerosol device comprising a canister equipped with a mechanical pump system and comprising a composition as defined in any of Claims 1 to 10 and a distribution system allowing the said composition to be delivered in mousse form.

## Patentansprüche

1. Zusammensetzung zum Färben von menschlichen Keratinfasern in Schaumform, umfassend:
(a) mindestens ein amphoteres Tensid;
(b) mindestens ein Alkanolamin;
(c) Ammoniumchlorid;
(d) mindestens ein Oxidationsmittel;
(e) mindestens eine Oxidationsfarbstoff-Vorstufe.

2. Zusammensetzung nach Anspruch 1, ferner umfassend ein anionisches Tensid.

3. Zusammensetzung nach Anspruch 1 oder 2, wobei das amphotere Tensid aus Alkyl(C₈-C₂₀)betainen, Sulfobetainen, Alkyl (C₈-C₂₀)amidoalkyl(C₁-C₆)betainen oder Alkyl (C₈-C₂₀)amidoalkyl(C₁-C₆)sulfobetainen, vorzugsweise Alkyl(C₈-C₂₀)amidoalkyl(C₁-C₆)betainen, ausgewählt ist.

4. Zusammensetzung nach einem der vorhergehenden Ansprüche, umfassend ein oder mehrere amphotere Tenside, ein oder mehrere nichtionische Tenside und ein oder mehrere anionische Tenside.

5. Zusammensetzung nach einem der vorhergehenden Ansprüche, wobei das Tensid bzw. die Tenside in einer Gesamtmenge von 0,1 bis 30 Gew.-%, vorzugsweise von 1 bis 20 Gew.-%, weiter bevorzugt von 1 bis 10 Gew.-%, bezogen auf das Gewicht der Zusammensetzung, vorliegt bzw. vorliegen.

6. Zusammensetzung nach einem der vorhergehenden Ansprüche, wobei es sich bei dem Alkanolamin um Monoethanolamin handelt.

7. Zusammensetzung nach einem der vorhergehenden Ansprüche, wobei die Menge an Alkanolamin von 0,01 bis 30 Gew.-%, vorzugsweise von 0,1 bis 20 Gew.-%, bezogen auf das Gewicht der Zusammensetzung, variiert.

8. Zusammensetzung nach einem der vorhergehenden Ansprüche, wobei die Menge an Ammoniumchlorid zwischen 0,01 bis 30 Gew.-%, vorzugsweise von 0,5 bis 10 Gew.-%, bezogen auf das Gewicht der Zusammensetzung, variiert.

9. Zusammensetzung nach einem der vorhergehenden Ansprüche, wobei es sich bei dem Oxidationsmittel um Wasserstoffperoxid handelt.

10. Zusammensetzung nach einem der vorhergehenden Ansprüche, umfassend eine oder mehrere Oxidationsbasen und einen oder mehrere Kuppler.

11. Aerosolvorrichtung mit einem Mittel zur Erzeugung einer Zusammensetzung gemäß einem der Ansprüche 1 bis 10 in Schaumform.

12. Aerosolvorrichtung nach Anspruch 11 mit einem Behälter mit 2 Kompartimenten oder mit zwei Behältern.

13. Nichtaerosolvorrichtung mit einem Kanister, der mit einem mechanischen Pumpsystem ausgestattet ist und eine Zusammensetzung gemäß einem der Ansprüche 1 bis 10 umfasst, und einem Verteilungssystem, das die Abgabe der Zusammensetzung in Schaumform erlaubt.

## Revendications

1. Composition de coloration des fibres kératiniques humaines sous forme de mousse comprenant :
(a) au moins un tensio-actif amphotère ;
(b) au moins une alcanolamine ;
(c) du chlorure d'ammonium ;
(d) au moins un agent oxydant ;
(e) au moins un précurseur de colorant d'oxydation.

2. Composition selon la revendication 1 comprenant de plus un tensioactif anionique.

3. Composition selon la revendication 1 ou 2 dans laquelle le tensioactif amphotère est choisi parmi les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes, de préférence les alkyl (C₈-C₂₀) amidoalkyl (C₁-C₆) bétaïnes.

4. Composition selon l'une quelconque des revendications précédentes comprenant un ou plusieurs tensioactifs amphotères, un ou plusieurs tensioactifs non ioniques et un ou plusieurs tensioactifs anioniques.

5. Composition selon l'une quelconque des revendications 1 à 4 dans laquelle le ou les tensioactifs sont présents en une quantité totale allant de 0,1 à 30 % en poids, de préférence de 1 à 20 %, mieux de 1 à 10% en poids par rapport au poids de la composition.

6. Composition selon l'une quelconque des revendications précédentes dans laquelle l'alcanolamine est la monoéthanolamine.

7. Composition selon l'une quelconque des revendications précédentes dans laquelle la teneur en alcanolamine varie de 0,01 à 30 % en poids, de préférence de 0,1 à 20 % en poids par rapport au poids de ladite composition.

8. Composition selon l'une quelconque des revendications précédentes dans laquelle la teneur en chlorure d'ammonium varie entre 0,01 à 30 % en poids, de préférence de 0,5 à 10 % en poids par rapport au poids de la composition.

9. Composition selon l'une quelconque des revendications précédentes dans laquelle l'agent oxydant est le peroxyde d'hydrogène.

10. Composition selon l'une quelconque des revendications précédentes, comprenant une ou plusieurs bases d'oxydation et un ou plusieurs coupleurs.

11. Dispositif aérosol comprenant un moyen de générer sous forme de mousse une composition telle que définie à l'une quelconque des revendications 1 à 10.

12. Dispositif aérosol selon la revendication 11 comprenant un seul récipient muni de 2 poches ou comprenant deux récipients.

13. Dispositif non aérosol comprenant un flacon équipé d'un système mécanique de pompage et comprenant une composition telle que définie à l'une quelconque des revendications 1 à 10 et un système de distribution permettant de la délivrer sous forme de mousse.
